(19) Europäisches Patentamt
European Patent Office
Office européen des brevets

(11) **EP 4 086 839 A1**

(12) **EUROPEAN PATENT APPLICATION**

(43) Date of publication:
**09.11.2022 Bulletin 2022/45**

(51) International Patent Classification (IPC):
**G06T 3/40** (2006.01)

(21) Application number: **21171997.6**

(22) Date of filing: **04.05.2021**

(52) Cooperative Patent Classification (CPC):
**G06T 3/4038; A61B 6/5241; G06T 7/33;**
G06T 2200/32; G06T 2207/10116;
G06T 2207/30012

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **SCHLUETER, Mathias**
**5656 AE Eindhoven (NL)**
• **MANKE, Dirk**
**5656 AE Eindhoven (NL)**
• **PRALOW, Thomas**
**5656 AE Eindhoven (NL)**

(74) Representative: **Philips Intellectual Property &
Standards
High Tech Campus 52
5656 AG Eindhoven (NL)**

(54) **STITCHING MULTIPLE IMAGES TO CREATE A PANORAMIC IMAGE**

(57)     There is provided a computer-implemented method for creating a panoramic radiographic image (600) of an object under examination (108). The method comprises: obtaining a stitching sequence comprising overlapping partial images of the object under examination; obtaining (401) data separating a marker region of the stitching sequence from an object region, the marker region depicting a stitching marker usable for image alignment; performing a first stitching operation, comprising performing image alignment on the stitching sequence using the marker as a matching feature and reconstructing (403A) therefrom a marker composite image (502); performing a second stitching operation, comprising performing image alignment on the stitching sequence using the object as a matching feature and reconstructing (403B) therefrom an object composite image (504); selecting image regions for inclusion in the panoramic radiographic image, comprising selecting (402A) the marker region of the marker composite image and selecting (402B) the object region of the object composite image; and combining (405) the selected image regions to form the panoramic radiographic image.

Fig. 4

## Description

FIELD OF THE INVENTION

The invention relates to methods and systems for creating panoramic radiographic images of objects under examination.

BACKGROUND OF THE INVENTION

Image stitching is a commonly used approach in radiography for anatomies that are larger than the size of the x-ray detector e.g., spine or leg exams. One possible acquisition approach is parallel stitching, in which the x-ray tube and detector are translated in parallel to the table between multiple exposures. The individual images are captured with a certain overlap, which is used to match the images automatically according to the content in the overlap area. Commonly, an x-ray opaque ruler is placed on the table close to the lateral image border. This ruler improves the robustness of the content-based automatic image registration in the overlap area. Furthermore, the ruler is used as a monitoring instrument for the correctness of the image registration. A resulting technical limitation of the parallel stitching approach is the occurrence of parallax errors, since the object under examination resides some distance above the ruler plane. Due to the parallax error, the matching result is inconsistent between different planes in the x-ray path. The matching result can be correct only for one image plane. If matching of the partial images is performed with respect to the ruler, parallax errors occur in the anatomy depending (among other factors) on the object-table-distance. Conversely, if matching is performed with respect to a representative anatomical feature, e.g. the spine plane, this results in double contours in the ruler, which thus cannot be used for validating the stitching result or for orthopedic measurements.

SUMMARY OF THE INVENTION

There is therefore a need for more effective avoidance of parallax error in panoramic radiographic images. This need is met by the subject-matter of the independent claims. Optional features are set forth by the dependent claims.

According to a first aspect, there is provided a computer-implemented method for creating a panoramic radiographic image of an object under examination. The method comprises: obtaining a stitching sequence comprising overlapping partial images of the object under examination; obtaining data separating a marker region of the stitching sequence from an object region, the marker region depicting a stitching marker usable for image alignment; performing a first stitching operation, comprising performing image alignment on the stitching sequence using the marker as a matching feature and reconstructing therefrom a marker composite image; performing a second stitching operation, comprising performing image alignment on the stitching sequence using the object as a matching feature and reconstructing therefrom an object composite image; selecting image regions for inclusion in the panoramic radiographic image, comprising selecting the marker region of the marker composite image and selecting the object region of the object composite image; and combining the selected image regions to form the panoramic radiographic image.

By reconstructing two separate composite images in different image planes, one in the marker plane (e.g. the ruler plane) and the other in the object plane (e.g. the anatomy plane), and selecting for inclusion in the final image pixels from the marker region of the marker composite image and those from the object region of the object composite image, parallax errors occurring in the marker may be avoided while parallax errors occurring in the object (e.g. anatomy) remain only minimal, i.e. those parallax errors unavoidably resulting from off-plane parts of the object.

The method may further comprise: obtaining data defining an object-marker distance; and, before forming the panoramic radiographic image, rescaling at least one of the marker region of the marker composite image and the object region of the object composite image to account for the object-marker distance. In this way, the marker may be moved into the representative object plane such that it can be used both for inspection of the matching result as well as a scale for orthopedic measurements. In one example, the rescaling may comprise applying a magnification factor to the marker region of the marker composite image, with the magnification factor being derived from the object-marker (and the X-ray-source-marker) distance, such that the marker may be virtually lifted (enlarged) into the object plane. Rescaling at least one of the marker region of the marker composite image and the object region of the object composite image may be understood as applying separate scaling factors to those regions. In particular, the rescaling is performed such that the said regions exhibit the same scale. In the case that the marker is positioned below the object, e.g. on the table, the object may be shown in the captured images at greater magnification than the marker. In this case, the marker region of the marker composite image may be magnified to match the scale of the object composite image. Equally, the object region of the object composite image may be reduced in scale to match that of the marker composite image. Alternatively, one region may be reduced in scale and the other enlarged, such that their scales match. In the case that the marker is positioned above the object, rescaling in the reverse direction to that described above may be performed. It will be understood that the rescaling may be performed in relation to the marker region of the marker composite image and/or the object region of the object composite image by rescaling the marker or object composite images as a whole, or alternatively only the said region may be rescaled.

The object-marker distance used for the rescaling may be obtained in a number of ways. In one example, obtaining the data defining an object-marker distance comprises receiving a user-input object-marker distance. Alternatively, the object-marker distance may be obtained via automatic feature-based image registration processes performed during stitching. Alternatively, the object-marker distance may be obtained during a calibration process.

In the case that the object-marker distance is input by the user or obtained during a calibration process, image registration during the second stitching operation may be performed using the so-obtained object-marker distance. In particular, performing the second stitching operation may comprise calculating the parallax error using the received object-marker distance, calculating at least one image displacement value for the second stitching operation based on the parallax error and on at least one image displacement value used in the first stitching operation, and implementing the image alignment using the calculated at least one image displacement value. The at least one image displacement value used in the first stitching operation may be obtained via an automatic image registration process.

In the case that the object-marker distance is obtained via automatic feature-based image registration processes performed during stitching, obtaining the data defining the object-marker distance may comprise calculating the object-marker distance based on at least one difference between a first set of image displacement values obtained via feature-based image registration performed in the first stitching operation and a second set of image displacement values obtained via feature-based image registration performed in the second stitching operation. Automatically determining the object-marker distance in such a way helps to avoid the cumbersome process of manually selecting a representative anatomy plane for an individual patient.

In the case that the object-marker distance is obtained during a calibration process, in which at least one of the partial images further depicts a calibration element positioned so as to define an object plane, obtaining the data defining the object-marker distance may comprise calculating the object-marker distance as the difference between a known detector-marker distance and a detector-object plane distance calculated based on a size of the calibration element as depicted in the at least one partial image.

The method may comprise determining an object height profile. In particular, the method may further comprise determining an object height profile based on differences between a first set of image displacement values obtained via feature-based image registration performed in the first stitching operation and a second set of image displacement values obtained via feature-based image registration performed in the second stitching operation. More particularly: the first stitching operation may comprise performing the feature-based image registration based on the marker and obtaining thereby the first set of image displacement values, while the second stitching operation comprises performing the feature-based image registration based on the object and obtaining thereby the second set of image displacement values. Each image displacement value represents the displacement used to register a respective pair of adjacent partial images or portions thereof. The method may then further comprise: calculating a set of difference values comprising differences between image displacement values in the first set and corresponding image displacement values in the second set, each difference value representing the parallax error at a corresponding longitudinal position along a marker plane in which the stitching marker resides; and calculating a set of local object-marker distances based on the difference values. The set of local object-marker distances represent an object height profile as a function of longitudinal position. As used herein, "longitudinal" refers to the direction in which the x-ray source and detector translate between image acquisitions. The term "corresponding" used in relation to the image displacement values means that two displacement values relate to the same pair of partial images or portions thereof, with one value having been obtained during the first stitching operation and the other during the second stitching operation. The method may then further comprise calculating an average object-marker distance based on the set of local object-marker distances and using the average object-marker distance when performing the rescaling.

The method may further comprise generating a 3D representation of a centerline of the object under examination based on the panoramic radiographic image and the object height profile and optionally taking (automatic) measurements of the centerline of the object using the 3D representation. Automatically-taken orthopedic spine measurements may provide clinical benefit.

Selecting and combining the image regions may be implemented in various ways. In one example, selecting the marker region of the marker composite image comprises cropping the partial images used in the first stitching operation and/or cropping the marker composite image to exclude pixels outside the marker region, while selecting the object region of the object composite image comprises cropping the partial images used in the second stitching operation and/or cropping the object composite image to exclude pixels outside the object region. Combining the selected image regions may then comprise merging the marker region of the marker composite image with the object region of the object composite image to form the panoramic radiographic image as a single image. It will be understood however that cropping is not necessary and that the image regions may be selected in other ways, for example using pixel-selection filters, or by voiding, greying out, or ignoring non-selected pixels. By "merging" is meant that the selected image regions are used to create a single image. In other examples, the selected image regions may be displayed side-by-side to create the impression of a single image.

"Panoramic" or "composite" as used herein refers to an image reconstructed by stitching together or mosaicking a plurality of partial images of the same scene. The generation of such a composite image from partial images is referred to herein as "reconstruction". "Partial image" refers to one image of a stitching sequence comprising multiple such partial images to be stitched together. The partial images or "stripes" partially overlap, allowing them to be matched and aligned in a stitching operation. The partial images may be captured by an imaging device whose field of view (FOV) is smaller than the region of interest (ROI) such that multiple partial images forming the stitching sequence are used to cover the ROI.

"Registration" as used herein refers to the determination of the displacement between adjacent partial images in the stitching sequence. Registration may be feature-based, meaning that it involves feature matching. More particularly, the registration process may find distinctive features (e.g. points, lines, and contours) in images and then match the distinctive features to establish correspondences between pairs of images. References to "matching features" herein are to be understood accordingly. Knowing the correspondence between a pair of images, a geometrical transformation may then be determined to map the target image to the reference image, thereby establishing point-by-point correspondence between the reference and target images. Feature detection algorithms (or keypoint or interest point detector algorithms) may be used to detect the distinctive features. "Image alignment" as used herein refers to the process of transforming one or both of the pair of images according to the determined transformation. References herein to performing registration with respect to a certain plane may be understood as matching in that plane.

By "stitching marker" is meant any marker usable for image alignment, preferably a continuous stitching marker which may be positioned substantially parallel to the direction of translation of the imaging device used to capture the stitching sequence, such that the marker, being radiopaque, appears in some or preferably all of the partial images to be stitched together, and more preferably in the overlapping portions of those partial images. One example of the stitching marker is the x-ray ruler described herein. Alternatively, multiple such stitching markers may be positioned to appear in respective overlap areas of the stitching sequence. The stitching marker may be placed in a marker plane which is separated from the representative object plane along the x-ray path.

By "(representative) object plane" or "(representative) anatomy plane" is meant a plane of interest extending through the object under examination which is intended to be free of parallax error, or afflicted only by minimal parallax error, for the purposes of medical imaging.

By "image region" is meant a group, set or subset of pixels within an image. The "marker region" or "ruler region" is an image region partially or wholly depicting the marker, or a portion of it sufficient for image registration purposes, regardless of whether the object is also depicted in that region. Data defining the marker region may be obtained during a manual or automatic feature detection process performed to detect the marker in the partial images. The "object region" or "anatomy region" may be understood as being the remainder of the image when the marker region is excluded, or as an image region partially or wholly depicting the object or anatomy.

The method of the first aspect may alternatively be described as a method for multiple plane parallel multi focus stitching in radiography.

According to a second aspect, there is provided an imaging method comprising: capturing a stitching sequence comprising overlapping partial images of an object under examination using a radiation-based imaging device; and performing the method of the first aspect using the captured stitching sequence to create a panoramic radiographic image of the object under examination.

According to a third aspect, there is provided a computing device comprising a processor configured to perform the method of the first aspect.

According to a fourth aspect, there is provided an imaging apparatus comprising: a radiation-based imaging device configured to capture a stitching sequence comprising overlapping partial images of an object under examination using; and the computing device of the third aspect.

According to a fifth aspect, there is provided a computer program product comprising instructions which, when executed by a computing device, enable or cause the computing device to perform the method of the first aspect.

According to a sixth aspect, there is provided a computer-readable medium comprising instructions which, when executed by a computing device, enable or cause the computing device to perform the method of the first aspect.

The invention may include one or more aspects, examples or features in isolation or combination whether or not specifically disclosed in that combination or in isolation. Any optional feature or sub-aspect of one of the above aspects applies as appropriate to any of the other aspects.

These and other aspects of the invention will be apparent from and elucidated with reference to the embodiments described hereinafter.

BRIEF DESCRIPTION OF THE DRAWINGS

A detailed description will now be given, by way of example only, with reference to the accompanying drawings, in which:-

Fig. 1 illustrates the parallax error occurring in an object plane in the overlap area of two neighboring partial images;

Fig. 2 illustrates the calculation of the parallax error $\Delta p$;

Fig. 3A shows an exemplary composite image produced when matching in a marker plane;

Fig. 3B shows an exemplary composite image produced when matching in an object plane;

Fig. 4 is a flowchart illustrating a method for creating a panoramic radiographic image of an object under examination;

Fig. 5 shows exemplary composite images produced using the method of Fig. 4;

Fig. 6 shows an exemplary panoramic image obtained by rescaling and merging the composite images of Fig. 5;

Fig. 7 shows an exemplary object height profile produced in conjunction with the method of Fig. 4; and

Fig. 8 illustrates a computing device that can be used in accordance with the systems and methods disclosed herein.

## DETAILED DESCRIPTION OF EMBODIMENTS

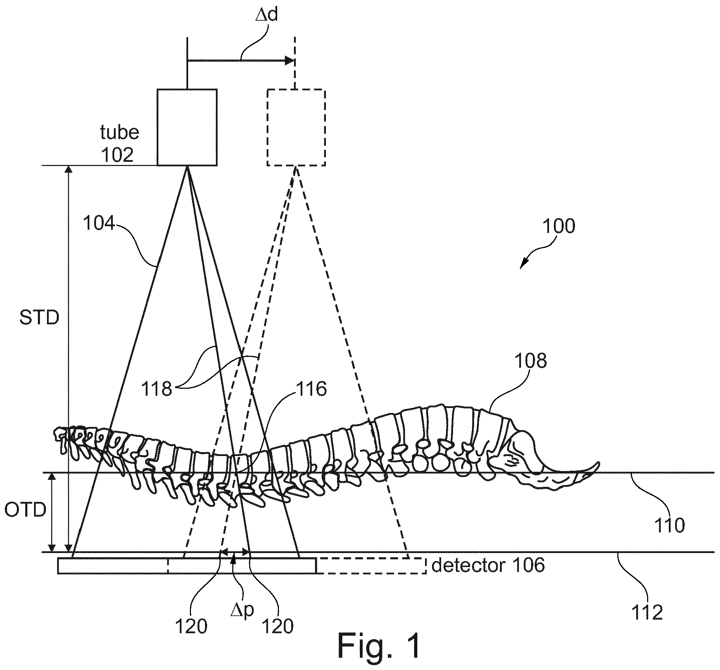

Fig. 1 illustrates an x-ray imaging system 100 in which an x-ray source 102 emits a conical beam 104 of radiation which is received by a detector 106. System 100 is used to capture one partial image at a first longitudinal position before both the x-ray source 102 and the detector 106 are translated by a distance $\Delta d$ and used to capture a second partial image at a second longitudinal position (as shown by the dashed lines in Fig. 1). Further partial images at further longitudinal positions may be captured in this way to obtain a stitching sequence. The focal spot distance $\Delta d$ may be calculated as $\Delta d = v/f$, where v is the detector velocity and $f$ is the frame rate. Object under examination 108 (in this example, a spine) represents the region of interest in the imaging field. The object 108 at least partially resides in a representative object plane 110. On a table (not shown) lies a stitching marker such as an x-ray ruler (not shown) extending in a marker plane 112. The location of the representative object plane 110 relative to the marker plane 112 is defined by an object-marker distance, described herein as the object-table distance OTD. The ruler appears in the overlap area of the partial images. In order to stitch the partial images, the ruler may be identified in the partial images and the partial images thereby registered and aligned so that the ruler accurately overlaps itself. If the registration is performed with respect to the ruler plane in this way, flat objects inside the ruler plane, e.g. the ruler, are stitched free of parallax error and will appear in focus in the composite image resulting from the stitching operation. However, the object 108 will appear blurred or doubled. In general, image alignment can only be achieved in a plane at a certain distance from the x-ray tube 102. This plane may be referred to as the registration plane. Stitching of images according to a certain registration plane causes objects out of that plane to appear blurred or doubled due to parallax error. Fig. 1 illustrates such a parallax error $\Delta p$ occurring in the overlap area. A certain point 116 of the object 108 in the object plane 110 is penetrated by x-rays in different directions 118 resulting in different projections 120 of the point 116 onto the overlap area. The resulting parallax error $\Delta p$ depends on the source-table distance (STD, the distance between the x-ray source 102 and the marker plane 112), the object-table distance (OTD), and the focal spot distance $\Delta d$ between consecutive images.

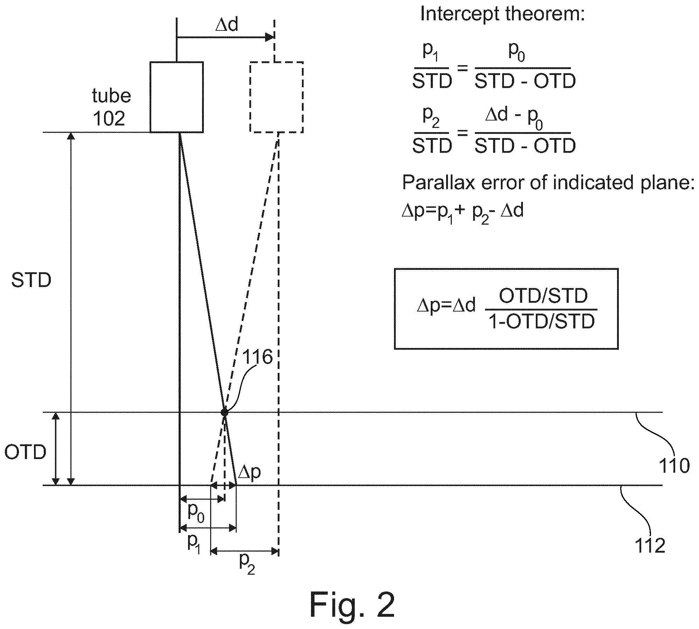

Fig. 2 illustrates the calculation of the parallax error $\Delta p$. The parallax error $\Delta p$ may be calculated as:

$$\Delta p = \Delta d \, \frac{OTD/STD}{1 - OTD/STD}. \qquad (1)$$

For example, the parallax error for $OTD$ = 10cm, $STD$ = 130cm, and $\Delta d$ =40mm is $\Delta p$ = 3.3mm, resulting in visible double contours in the composite image.

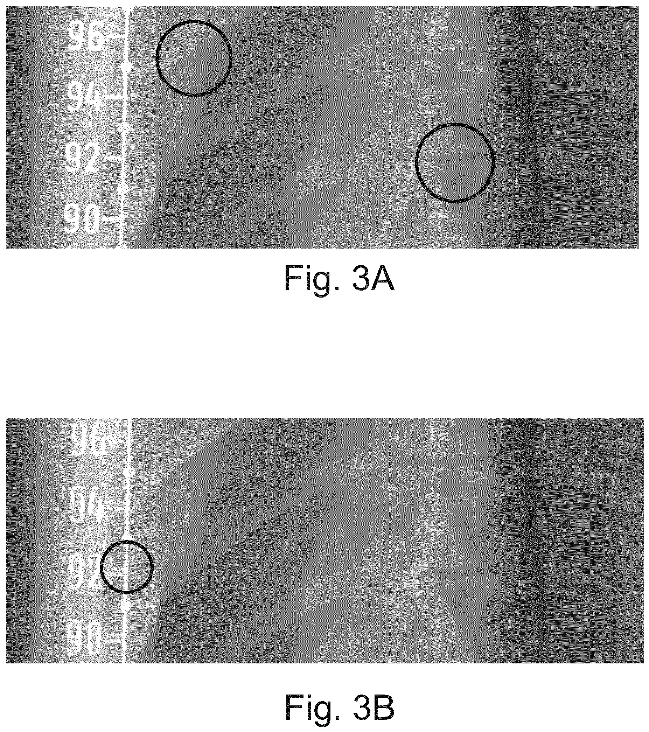

Fig. 3A shows an exemplary composite image obtained when registration is performed with respect to the marker plane 112. The circles indicate double contours in the object due to the parallax error.

Conversely, Fig. 3B illustrates the scenario in which registration is performed with respect to the representative object plane 110, resulting in double contours in the ruler. In consequence, the ruler cannot be used for visual inspection of the automatic stitching result. Additionally, with respect to a detector plane, the ruler is less magnified than the object. Therefore, the ruler cannot be used as a scale for orthopedic measurements. Furthermore, the object 108 does not reside entirely within the object plane 110. Thus, there will remain some intra-object parallax error even if registration is performed with respect to the representative object plane 110.

According to the present disclosure, there is therefore provided a multiple plane parallel multi focus stitching method involving generating two composite image reconstructions with respect to the marker plane and object plane, respectively, and merging them into one image. This is based on the recognition that, for stitching acquisitions, the ruler region is typically separated from the anatomy region.

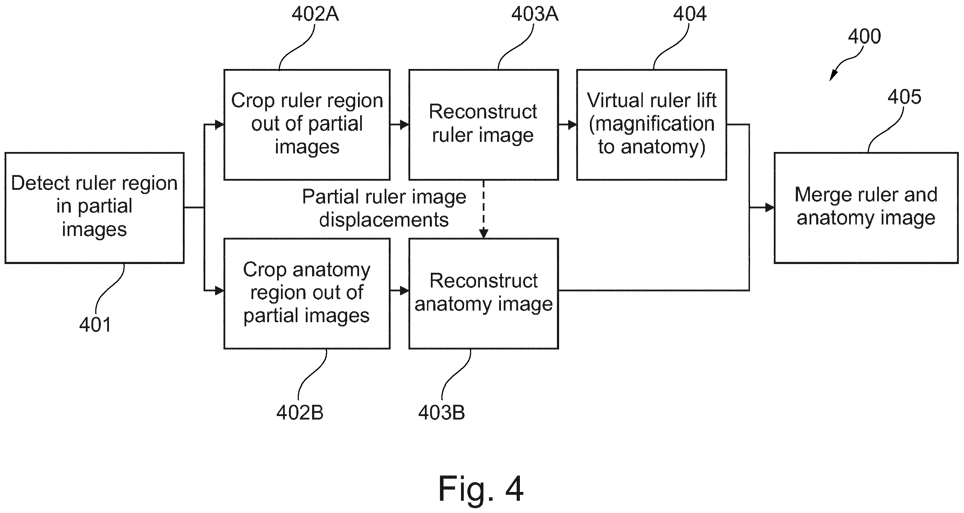

Fig. 4 is a flowchart illustrating a method 400 for creating a panoramic radiographic image of an object under examination from partial images captured within a multi focus parallel stitching sequence.

Step 401 comprises detecting the ruler region within the partial images. The ruler region can be detected either automatically or manually.

Step 402A comprises cropping the ruler region out of the partial images to retain only the ruler region.

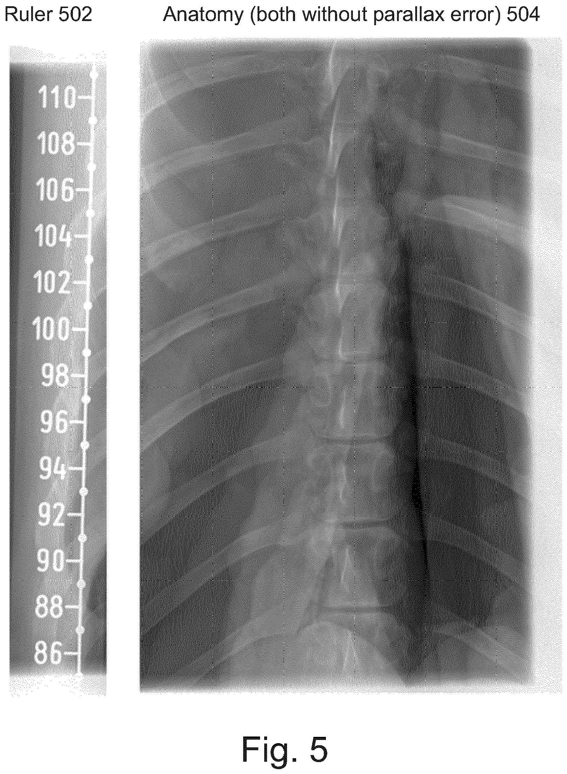

Step 403A comprises reconstructing a ruler composite image without the anatomy by stitching the cropped partial images containing only the ruler region. Fig. 5 shows an exemplary ruler composite image 502 on the left-hand side. During step 403A, a set of longitu-

dinal partial image displacements $t_r$ obtained during the reconstruction of the ruler composite image 502 is produced.

Step 402B comprises cropping the anatomy out of the partial images as captured to exclude the ruler region from the partial images.

Step 403B comprises reconstructing an anatomy composite image by stitching the cropped partial images containing only the anatomy region. Fig. 5 shows an exemplary anatomy composite image 504 on the right-hand side. The anatomy composite image can be reconstructed either by automatic registration of the anatomy or by subtracting the parallax error $\Delta p$ from the longitudinal partial image displacements $t_r$ obtained during the reconstruction of the ruler composite image ($t_a = t_r\text{-}\Delta p$). The displacements $t_a$ are then used for the reconstruction of the anatomy composite image. The parallax error is determined with respect to the height of the representative anatomy plane, i.e. OTD, using equation (1) given above. The representative anatomy plane at height OTD can be configured by the user. Thus, a parallax error correction is applied to the partial image displacements with respect to the OTD.

Step 404 comprises performing a virtual lift of the ruler to the representative anatomy plane by applying a magnification factor m = STD / (STD - OTD) to the ruler composite image.

Step 405 comprises merging the magnified ruler composite image and the anatomy composite image to form one panoramic image.

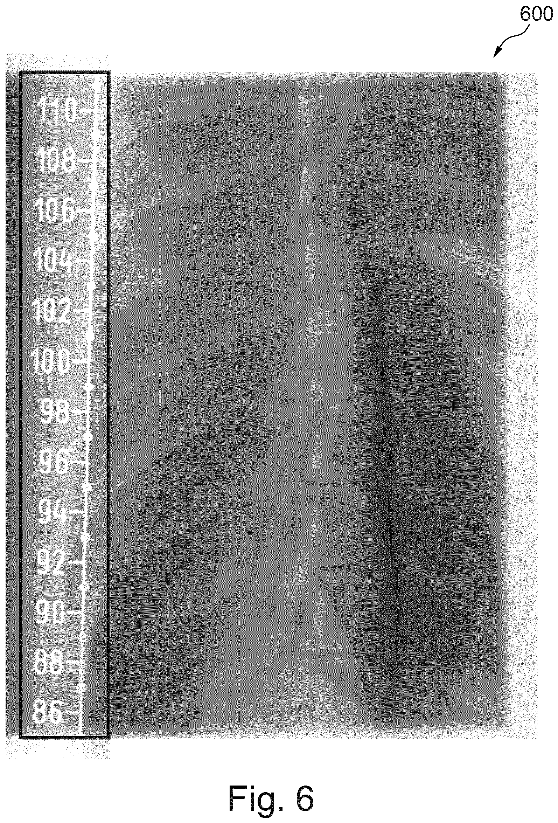

Fig. 6 shows an exemplary panoramic image 600 obtained via the merging of the magnified ruler composite image 502 of Fig. 5 and the anatomy composite image 504. In this example STD = 120 cm and OTD = 10 cm leading to a magnification factor of 1.09 (9%).

With the above-described method 400, the ruler as well as the representative anatomy plane are displayed substantially free of parallax error within one image. Furthermore, the ruler can be used as a scale for orthopedic measurements, because it has the same magnification in the detector plane as the representative anatomy plane.

The method 400 may be further used to determine an anatomy height profile from the anatomy-based stitching result. In particular, if, for each pair of adjacent partial images, the registration of the ruler and anatomy is performed independently and image-based, two sets of partial image displacements are obtained, namely $\{t_r\}$ and $\{t_a\}$. If a longitudinal position, x, is assigned to each pair of adjacent partial images, the difference between the displacement of the anatomy and ruler registration at a certain longitudinal position x is equal to the local parallax error $\Delta p(x)$ of the anatomy with respect to the ruler plane at that longitudinal position x:

$$\Delta p(x) = t_r(x) - t_a(x).$$

Thus, by inverting the parallax error equation (1), an anatomy height profile with respect to the ruler plane may be constructed as a function of the longitudinal position:

$$OTD(x) = STD \cdot \frac{\Delta p(x)}{\Delta p(x) + \Delta d}. \qquad (2)$$

For spine stitching, the anatomy height profile is typically a curve, since the spine is curved with respect to the detector plane (see Fig. 1).

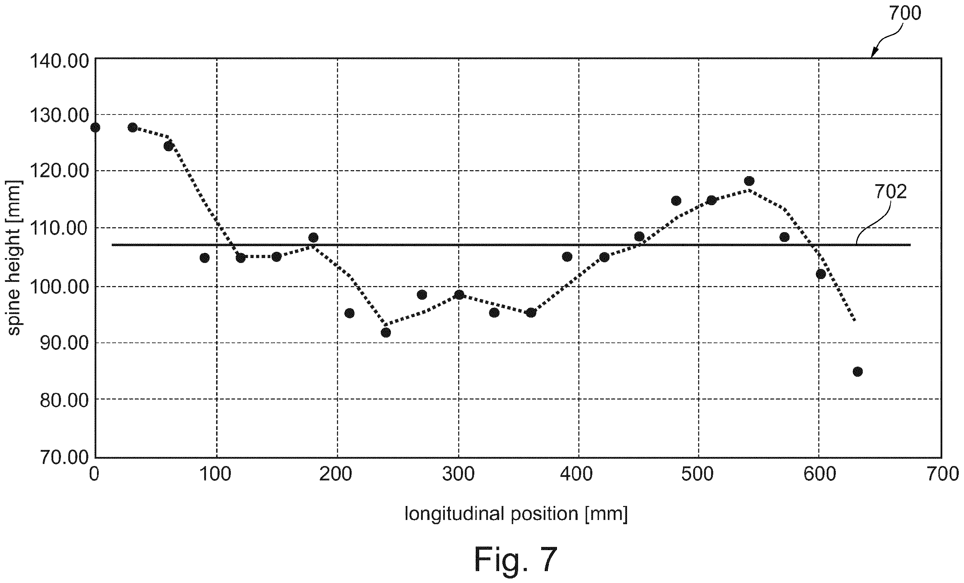

Fig. 7 shows one example of a spine height profile 700 showing spine height as a function of longitudinal position.

From the anatomy height profile, an average height can be calculated and taken as the height of the representative anatomy plane. This height can be used for the virtual ruler lift described above. Line 702 in Fig. 7 represents the average of the height profile.

Furthermore, the anatomy height profile provides an approximation of the local anteroposterior (AP) position of the spine. In combination with the centerline taken from the panoramic (AP) image of the spine, a 3D representation of the spine (i.e. its curvature) can be derived. This 3D representation may be used for (automatic) orthopedic spine measurements.

The determination of an anatomy height profile from stitching transformations requires a certain amount of anatomical structure in the lateral direction. While this is the case for the spine, it is hardly the case for legs. For long leg stitching operations, the representative anatomy plane may be determined manually or using a calibration sphere of known diameter which is positioned next to the patient in the plane of the relevant anatomy e.g. femur bone. The calibration sphere thus defines the virtual object plane, which should be reconstructed parallax-error-free. The distance of the calibration sphere from the detector (OID) is calculated from the size of the sphere in an individual image. The OTD can then be calculated based on a known table-detector distance (TID) as OTD = OID -TID. As described above, the parallax error can be calculated with respect to OTD and the parallax error in the representative anatomy plane can be corrected. The ruler may then be virtually lifted in the above-described manner using the calculated OTD.

The invention can be applied for all diagnostic and interventional radio-fluoroscopy and radiography systems, e.g. Philips CombiDiagnost, ProxiDiagnost, DigitalDiagnost C90, and image-guided mobile surgery systems.

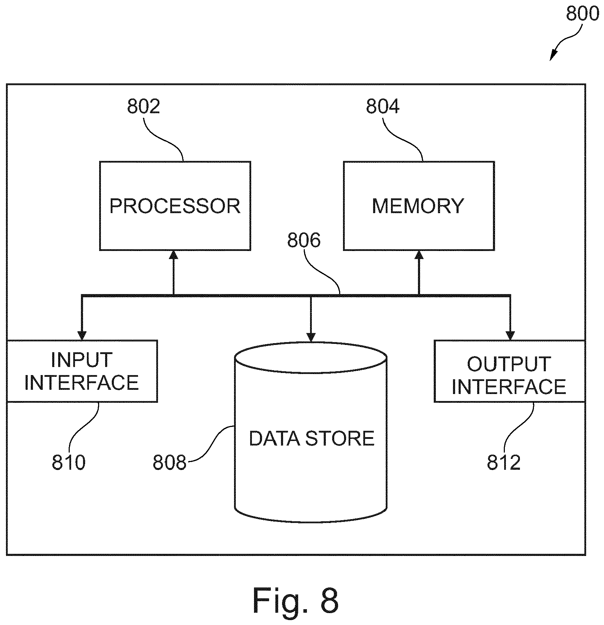

Referring now to Fig. 8, a high-level illustration of an exemplary computing device 800 that can be used in accordance with the systems and methodologies disclosed herein is illustrated. The computing device 800 includes at least one processor 802 that executes instructions that are stored in a memory 804. The instructions may be, for instance, instructions for implementing functionality described as being carried out by one or more components discussed above or instructions for implementing one or more of the methods described above. The processor 802 may access the memory 804 by way of a system bus 806. In addition to storing executable instructions, the memory 804 may also store conversational inputs, scores assigned to the conversational inputs, etc.

The computing device 800 additionally includes a data store 808 that is accessible by the processor 802 by way of the system bus 806. The data store 808 may include executable instructions, log data, etc. The computing device 800 also includes an input interface 810 that allows external devices to communicate with the computing device 800. For instance, the input interface 810 may be used to receive instructions from an external computer device, from a user, etc. The computing device 800 also includes an output interface 812 that interfaces the computing device 800 with one or more external devices. For example, the computing device 800 may display text, images, etc. by way of the output interface 812.

It is contemplated that the external devices that communicate with the computing device 800 via the input interface 810 and the output interface 812 can be included in an environment that provides substantially any type of user interface with which a user can interact. Examples of user interface types include graphical user interfaces, natural user interfaces, and so forth. For instance, a graphical user interface may accept input from a user employing input device(s) such as a keyboard, mouse, remote control, or the like and provide output on an output device such as a display. Further, a natural user interface may enable a user to interact with the computing device 800 in a manner free from constraints imposed by input device such as keyboards, mice, remote controls, and the like. Rather, a natural user interface can rely on speech recognition, touch and stylus recognition, gesture recognition both on screen and adjacent to the screen, air gestures, head and eye tracking, voice and speech, vision, touch, gestures, machine intelligence, and so forth.

Additionally, while illustrated as a single system, it is to be understood that the computing device 800 may be a distributed system. Thus, for instance, several devices may be in communication by way of a network connection and may collectively perform tasks described as being performed by the computing device 800.

Various functions described herein can be implemented in hardware, software, or any combination thereof. If implemented in software, the functions can be stored on or transmitted over as one or more instructions or code on a computer-readable medium. Computer-readable media includes computer-readable storage media. A computer-readable storage media can be any available storage media that can be accessed by a computer. By way of example, and not limitation, such computer-readable storage media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Disk and disc, as used herein, include compact disc (CD), laser disc, optical disc, digital versatile disc (DVD), floppy disk, and Blu-ray disc (BD), where disks usually reproduce data magnetically and discs usually reproduce data optically with lasers. Further, a propagated signal is not included within the scope of computer-readable storage media. Computer-readable media also includes communication media including any medium that facilitates transfer of a computer program from one place to another. A connection, for instance, can be a communication medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio and microwave are included in the definition of communication medium. Combinations of the above should also be included within the scope of computer-readable media.

Alternatively, or in addition, the functionally described herein can be performed, at least in part, by one or more hardware logic components. For example, and without limitation, illustrative types of hardware logic components that can be used include Field-programmable Gate Arrays (FPGAs), Program-specific Integrated Circuits (ASICs), Program-specific Standard Products (ASSPs), System-on-a-chip systems (SOCs), Complex Programmable Logic Devices (CPLDs), etc.

It will be appreciated that the aforementioned circuitry may have other functions in addition to the mentioned functions, and that these functions may be performed by the same circuit.

The applicant hereby discloses in isolation each individual feature described herein and any combination of two or more such features, to the extent that such features or combinations are capable of being carried out based on the present specification as a whole in the light of the common general knowledge of a person skilled in the art, irrespective of whether such features or combinations of features solve any problems disclosed herein, and without limitation to the scope of the claims. The applicant indicates that aspects of the present invention may consist of any such individual feature or combination of features.

It has to be noted that embodiments of the invention are described with reference to different categories. In particular, some examples are described with reference to methods whereas others are described with reference to apparatus. However, a person skilled in the art will gather from the description that, unless otherwise notified, in addition to any combination of features belonging to one category, also any combination between features relating to different category is considered to be disclosed by this application. However, all features can be combined to provide synergetic effects that are more than the simple summation of the features.

While the invention has been illustrated and described in detail in the drawings and foregoing description, such illustration and description are to be considered exemplary and not restrictive. The invention is not limited to the disclosed embodiments. Other variations to the disclosed embodiments can be understood and effected by those skilled in the art, from a study of the drawings, the disclosure, and the appended claims.

The word "comprising" does not exclude other elements or steps.

The indefinite article "a" or "an" does not exclude a plurality. In addition, the articles "a" and "an" as used herein should generally be construed to mean "one or more" unless specified otherwise or clear from the context to be directed to a singular form.

A single processor or other unit may fulfil the functions of several items recited in the claims.

The mere fact that certain measures are recited in mutually different dependent claims does not indicate that a combination of these measures cannot be used advantageously.

A computer program may be stored/distributed on a suitable medium, such as an optical storage medium or a solid-state medium supplied together with or as part of other hardware, but may also be distributed in other forms, such as via the internet or other wired or wireless communications systems.

Any reference signs in the claims should not be construed as limiting the scope.

Unless specified otherwise, or clear from the context, the phrase "A and/or B" as used herein is intended to mean all possible permutations of one or more of the listed items. That is, the phrase "X comprises A and/or B" is satisfied by any of the following instances: X comprises A; X comprises B; or X comprises both A and B.

**Claims**

1. A computer-implemented method for creating a panoramic radiographic image (600) of an object under examination (108), the method comprising:

   obtaining a stitching sequence comprising overlapping partial images of the object under examination;
   obtaining (401) data separating a marker region of the stitching sequence from an object region, the marker region depicting a stitching marker usable for image alignment;
   performing a first stitching operation, comprising performing image alignment on the stitching sequence using the marker as a matching feature and reconstructing (403A) therefrom a marker composite image (502);
   performing a second stitching operation, comprising performing image alignment on the stitching sequence using the object as a matching feature and reconstructing (403B) therefrom an object composite image (504);
   selecting image regions for inclusion in the panoramic radiographic image, comprising selecting (402A) the marker region of the marker composite image and selecting (402B) the object region of the object composite image; and
   combining (405) the selected image regions to form the panoramic radiographic image.

2. The method of claim 1, further comprising:

   obtaining data defining an object-marker distance (OTD); and,
   before forming the panoramic radiographic image (600), rescaling (404) at least one of the marker region of the marker composite image (502) and the object region of the object composite image (504) to account for the object-marker distance.

3. The method of claim 2, wherein the rescaling comprises applying (404) a magnification factor to the marker region of the marker composite image (502), the magnification factor being derived from the object-marker distance (OTD).

4. The method of any claim 2 or 3, wherein obtaining the data defining an object-marker distance (OTD) comprises receiving the object-marker distance from the user or from a calibration process, and wherein performing the second stitching operation comprises calculating the parallax error ($\Delta p$) using the received object-marker distance, calculating at least one image displacement value for the second stitching operation based on the parallax error and on at least one image displacement value used in the first stitching operation, and implementing the image alignment using the calculated at least one image displacement value.

5. The method of any preceding claim, further comprising determining an object height profile (700) based on differences between a first set of image displacement values obtained via feature-based image registration performed in the first stitching operation and a second set of image displacement values obtained via feature-based image registration performed in the second stitching operation.

6. The method of any of claims 2-5, wherein obtaining the data defining the object-marker distance (OTD) comprises calculating the object-marker distance based on at least one difference between a first set of image displacement values obtained via feature-based image registration performed in the first stitching operation and a second set of image displacement values obtained via feature-based image registration performed in the second stitching operation.

7. The method of claim 5 or 6,
wherein the first stitching operation comprises performing the feature-based image registration based on the marker and obtaining thereby the first set of image displacement values,

   wherein the second stitching operation comprises performing the feature-based image registration based on the object (108) and obtaining thereby the second set of image displacement values,
   each image displacement value representing the displacement used to register a respective pair of adjacent partial images or portions thereof, the method further comprising:

      calculating a set of difference values comprising differences between image displacement values in the first set and corresponding image displacement values in the second set, each difference value representing the parallax error at a corresponding longitudinal position along a marker plane (112) in which the stitching marker resides; and
      calculating a set of local object-marker distances based on the difference values, the set of local object-marker distances representing an object height profile (700) as a function of longitudinal position.

8. The method of claims 6 and 7, further comprising calculating an average object-marker distance (702) based on the set of local object-marker distances and using the average object-marker distance when performing the rescaling.

9. The method of claim 5, 7 or 8, further comprising generating a 3D representation of a centerline of the object under examination (108) based on the panoramic radiographic image (600) and the object height profile (700) and optionally taking measurements of the centerline of the object using the 3D representation.

10. The method of claim 2, wherein at least one of the partial images further depicts a calibration element positioned so as to define an object plane (110), and wherein obtaining the data defining the object-marker distance (OTD) comprises calculating the object-marker distance as the difference between a known detector-marker distance (STD) and a detector-object plane distance calculated based on a size of the calibration element as depicted in the at least one partial image.

11. The method of any preceding claim,
wherein selecting the marker region of the marker composite image (502) comprises cropping (402A) the partial images used in the first stitching operation and/or cropping the marker composite image to exclude pixels outside the marker region,
wherein selecting the object region of the object composite image (504) comprises cropping (403A) the partial images used in the second stitching operation and/or cropping the object composite image to exclude pixels outside the object region, and
wherein combining the selected image regions comprises merging (405) the marker region of the marker composite image with the object region of the object composite image to form the panoramic radiographic image (600) as a single image.

12. An imaging method comprising:

   capturing a stitching sequence comprising overlapping partial images of an object under examination using a radiation-based imaging device; and
   performing the method of any preceding claim using the captured stitching sequence to create a panoramic radiographic image of the object under examination.

13. A computing device (800) comprising a processor (802) configured to perform the method of any of claims 1-11.

14. An imaging apparatus comprising:

   a radiation-based imaging device configured to capture a stitching sequence comprising overlapping partial images of an object under examination using; and
   the computing device of claim 13.

15. A computer-readable medium (804, 808) comprising instructions which, when executed by a computing device (800), enable the computing device to carry out the method of any of claims 1-11.

Fig. 1

EP 4 086 839 A1

Intercept theorem:

$$\frac{p_1}{STD} = \frac{p_0}{STD - OTD}$$

$$\frac{p_2}{STD} = \frac{\Delta d - p_0}{STD - OTD}$$

Parallax error of indicated plane:

$$\Delta p = p_1 + p_2 - \Delta d$$

$$\Delta p = \Delta d \, \frac{OTD/STD}{1 - OTD/STD}$$

$\Delta d$

tube
102

STD

OTD

116

$\Delta p$

$p_0$

$p_1$

$p_2$

110

112

Fig. 2

Fig. 3A

Fig. 3B

Fig. 4

Ruler 502    Anatomy (both without parallax error) 504

Fig. 5

Fig. 6

Fig. 7

EP 4 086 839 A1

Fig. 8

Europäisches
Patentamt
European
Patent Office
Office européen
des brevets

**EUROPEAN SEARCH REPORT**

Application Number

EP 21 17 1997

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/188726 A1 (NATHANIEL RAM [IL] ET AL) 4 August 2011 (2011-08-04)<br>* paragraph [0006] - paragraph [0007] *<br>* paragraph [0024] *<br>* paragraph [0037] *<br>* paragraph [0096] - paragraph [0099]; claim 1; figures 1-2 * | 1-8, 10-15 | INV.<br>G06T3/40 |
| A | JOSKOWICZ L ET AL: "Long Bone Panoramas From Fluoroscopic X-Ray Images", IEEE TRANSACTIONS ON MEDICAL IMAGING,, vol. 23, no. 1, 1 January 2004 (2004-01-01), pages 26-35, XP011104510,<br>* page 31, right-hand column, paragraph 2 - page 32, right-hand column, paragraph 1 * | 1-15 | |
| A | GUAN SHAO-YA ET AL: "A Review of Point Feature Based Medical Image Registration", CHINESE JOURNAL OF MECHANICAL ENGINEERING, vol. 31, no. 1, 24 August 2018 (2018-08-24), XP55847755,<br>* page 3, left-hand column, paragraph 3 - right-hand column, paragraph 1 *<br>* page 14, left-hand column, paragraph 3 * | 1-15 | TECHNICAL FIELDS SEARCHED (IPC)<br><br>G06T |

The present search report has been drawn up for all claims

| Place of search | Date of completion of the search | Examiner |
|---|---|---|
| Munich | 8 October 2021 | Pierfederici, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 086 839 A1**

## ANNEX TO THE EUROPEAN SEARCH REPORT
## ON EUROPEAN PATENT APPLICATION NO.

EP 21 17 1997

This annex lists the patent family members relating to the patent documents cited in the above-mentioned European search report.
The members are as contained in the European Patent Office EDP file on
The European Patent Office is in no way liable for these particulars which are merely given for the purpose of information.

08-10-2021

| Patent document cited in search report | | | Publication date | Patent family member(s) | | | Publication date |
|---|---|---|---|---|---|---|---|
| US 2011188726 | A1 | | 04-08-2011 | CN | 102124320 | A | 13-07-2011 |
| | | | | EP | 2310839 | A1 | 20-04-2011 |
| | | | | US | 2011188726 | A1 | 04-08-2011 |
| | | | | WO | 2009153789 | A1 | 23-12-2009 |

EPO FORM P0459

For more details about this annex : see Official Journal of the European Patent Office, No. 12/82